# FASCICULE DE BREVET EUROPEEN

(11) **EP 1 342 471 B1**
(45) Date de publication et mention de la délivrance du brevet: **08.06.2005**
(21) Numéro de dépôt: 03290274.4
(22) Date de dépôt: 04.02.2003
(51) Int. Cl.: A61K 9/51, A61K 7/00

(54) **Nanocapsules a base de polyester polyol, et compositions cosmetiques ou dermatologiques les contenant**
Nanokapseln auf Basis von Polyolpolyestern und diese enthaltende kosmetische oder dermatologische Zusammensetzungen
Nanocapsules based on polyol polyester, and cosmetic and dermatological compositions containing same

(30) Priorité: 22.02.2002 FR 0202290
(43) Date de publication de la demande: 10.09.2003
(73) Titulaire: L'OREAL, 75008 Paris (FR)
(72) Inventeur: Simonnet, Jean-Thierry, 75011 Paris (FR); Guerin, Gilles, 95600 Eaubonne (FR)
(74) Mandataire: Rasson, Catherine

(56) Documents cités:
- EP-A- 1 034 839
- FR-A- 2 787 729
- FR-A- 2 787 730

## Description

La présente invention se rapporte à des nanocapsules constituées d'un coeur lipidique formant ou contenant un actif lipophile et d'une enveloppe continue insoluble dans l'eau, comprenant au moins un polyester polyol obtenu par polycondensation d'un acide dicarboxylique aliphatique avec au moins deux alcane diols ou avec au moins un alcane-diol et au moins un hydroxyalkyl alcane-diol. L'invention se rapporte aussi au procédé de préparation de ces nanocapsules, ainsi qu'aux compositions cosmétiques ou dermatologiques contenant ces nanocapsules et à leurs applications dans le domaine cosmétique ou dermatologique.

L'encapsulation ou l'absorption de principes actifs liphophiles dans des particules de dimensions submicroniques (inférieures à 1 µm) est connue depuis plusieurs années et est largement utilisée en particulier dans les domaines cosmétique et dermatologique. En effet, ces particules appelées nanoparticules sont capables de traverser les couches superficielles du stratum corneum et de pénétrer dans les couches supérieures de l'épiderme vivant pour y libérer le principe actif. Cette pénétration dans des couches plus profondes élargit l'espace d'action des principes actifs et les met à l'abri d'une élimination rapide par simple frottement.

Le terme de "nanoparticules" englobe principalement deux systèmes différents : des "nanosphères" constituées d'une matrice polymérique poreuse dans laquelle le principe actif est absorbé et/ou adsorbé, ainsi que des "nanocapsules" ayant une structure de type noyau-enveloppe, c'est-à-dire une structure constituée d'un coeur lipidique formant ou contenant le principe actif, lequel coeur est encapsulé dans une enveloppe protectrice continue insoluble dans l'eau. La présente invention concerne uniquement ce deuxième type vésiculaire de nanoparticules, c'est-à-dire des nanocapsules à noyau lipidique entouré d'une membrane polymère.

L'encapsulation de principes actifs dans des capsules de taille submicronique permet, il est vrai, de transporter les molécules actives plus profondément dans la peau mais elle n'assure pas toujours une stabilité suffisante de l'actif vis-à-vis des conditions physico-chimiques environnantes, notamment pour les actifs sensibles à l'environnement (lumière, oxygène, chaleur) et notamment sensibles l'oxydation et à l'hydrolyse. Le problème de l'instabilité des actifs sensibles à l'oxydation et à l'hydrolyse en milieu aqueux se pose en particulier quand on veut les introduire dans un milieu contenant de l'eau et/ou les manipuler à l'air libre. Or, pour diverses raisons liées en particulier à un meilleur confort d'utilisation (douceur, émollience et autres), les compositions cosmétiques ou dermatologiques, notamment les compositions de soin de la peau, se présentent le plus souvent sous la forme d'une émulsion constituée d'une phase aqueuse et d'une phase huileuse, soit sous forme d'émulsion huile-dans-eau (H/E) soit sous forme d'émulsion eau-dans-huile (E/H). Les actifs lipophiles sont généralement introduits dans la phase huileuse de l'émulsion mais du fait de la présence d'une phase aqueuse, ils ont tendance à être destabilisés et donc à perdre de leur activité, ce qui va à l'encontre du but recherché.

Comme actifs lipophiles ayant tendance à se déstabiliser en milieu aqueux, on peut citer par exemple le rétinol et ses dérivés et notamment ses esters, plus particulièrement les esters à chaîne courte, les esters ayant d'autant plus tendance à s'hydrolyser que la chaîne alkyle est courte (C2 à C10).

Une solution pour stabiliser le rétinol a consisté à l'encapsuler dans des nanocapsules à base de poly(alkylène adipate), comme décrit dans le document FR-A-2,787,730, le terme de poly(alkylène adipate) englobant les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols.

Toutefois, seuls ont été exemplifiés dans ce document, les polymères obtenus à partir d'acide adipique et d'un alcane-diol et ayant une masse molaire de 10 000, et il a été observé que ces nanocapsules ne permettent pas de stabiliser suffisamment les esters de rétinol, notamment ceux sensibles à l'hydrolyse en milieu aqueux, c'est-à-dire ceux ayant une chaîne alkyle courte, tels que par exemple le propionate de rétinol.

Il subsiste donc le besoin d'une composition permettant d'obtenir une bonne stabilité des actifs sensibles à un environnement oxydant et/ou aqueux, tels que le rétinol et de ses dérivés, notamment ses esters sensibles à l'hydrolyse.

La présente demande répond à ce besoin. En effet, la demanderesse a découvert que l'encapsulation dans des nanocapsules à base d'un type de polyester polyol particulier permettait d'améliorer de manière spectaculaire la stabilité des actifs lipophiles sensibles à l'oxydation et à l'hydrolyse, notamment des esters de rétinol.

Ainsi, comme on le verra dans les exemples ci-après, l'encapsulation du propionate de rétinol dans les nanocapsules selon l'invention confère à cette molécule active une stabilité satisfaisante, à savoir une perte d'activité de seulement 12 à 15 % après un mois de conservation à 45 °C, alors que, dans des conditions équivalentes, cette même molécule encapsulée dans des polymères de l'art antérieur [poly(éthylène)adipate] présente une perte d'activité de 31 %.

L'invention a donc pour objet une nanocapsule constituée :
- d'un coeur lipidique formant ou contenant au moins un actif lipophile et
- d'une enveloppe polymérique continue insoluble dans l'eau, comprenant au moins un polyester polyol obtenu par polycondensation d'au moins un acide dicarboxylique aliphatique avec au moins deux alcane-diols ou avec au moins un alcane-diol et au moins un hydroxyalkyl alcane-diol comportant éventuellement une chaîne alkyle, le polyester polyol ayant une masse molaire moyenne en poids inférieure à 5000.

Les nanocapsules obtenues selon l'invention permettent d'obtenir d'excellents résultats de stabilité pour les actifs sensibles, notamment pour les esters de rétinol en C1-C6, molécules très sensibles à l'hydrolyse, tels que l'acétate de rétinol et le propionate de rétinol.

Les polyesters polyols utilisables pour former l'enveloppe des nanocapsules sont des copolymères obtenus par polycondensation d'au moins un acide dicarboxylique aliphatique, avec au moins deux alcane-diols, ou avec au moins un alcane-diol et au moins un hydroxyalkyl alcane-diol), et, éventuellement, une faible proportion de triols.

L'acide dicarboxylique aliphatique utilisé pour la préparation desdits polyesters polyols peut être choisi par exemple parmi l'acide malonique, l'acide succinique, l'acide glutarique, l'acide adipique (ou acide hexane-1,6-dioïque), l'acide pinélique, l'acide sébacique, l'acide azélaique et leurs mélanges. Selon un mode préféré de réalisation de l'invention, l'acide dicarboxylique est l'acide adipique.

Les alcane-diols utilisés pour la préparation des polyesters polyols sont de préférence choisis parmi des alcane-diols à chaîne linéaire ou ramifiée comportant de 2 à 20 atomes de carbone, et de préférence de 2 à 10 atomes de carbone. Ils peuvent être choisis notamment parmi l'éthylèneglycol, le propylèneglycol, le 1,3-propanediol, le 1,4-butanediol, le 1,5-pentanediol, le 1,6-hexanediol, le néopentylglycol et leurs mélanges. Selon un mode préféré de réalisation de l'invention, l'alcane-diol est choisi parmi le 1,4-butanediol, le 1,6-hexane diol et leurs mélanges. De manière encore plus préférée, l'alcane-diol est le 1,4-butanediol.

On entend dans la présente demande par « hydroxyalkyl alcane-diols comportant éventuellement une chaîne alkyle » des alcane-diols comportant au moins un groupe hydroxyalkyle, et pouvant en outre comporter une chaîne alkyle, où le groupe hydroxyalkyle et la chaîne alkyle sont, indépendamment l'un de l'autre, des chaînes saturées, linéaires ou ramifiées, comportant de 1 à 10 atomes de carbone. Les hydroxyalkyl alcane-diols utilisables pour former les polyesters polyols de la présente invention peuvent être choisis par exemple parmi les 2-alkyl-2-(hydroxyalkyl)-1,3-propanediol, où le groupe hydroxyalkyle et la chaîne alkyle comportent, indépendamment l'un de l'autre, de 1 à 10 atomes de carbone, tels que par exemple le 2-éthyl-2-(hydroxyméthyl)-1,3-propanediol et le 2-méthyl-2-(hydroxyméthyl)-1,3-propanediol ; les 2-(hydroxyalkyl)-1,3-propanediol où le groupe hydroxyalkyle comporte de 1 à 10 atomes de carbone ; et leurs mélanges.

Selon un mode préféré de réalisation de invention, on utilise comme hydroxyalkyl alcane-diol, le 2-éthyl-2-(hydroxyméthyl)-1,3-propanediol.

Comme indiqué ci-dessus, les polyesters polyols utilisés pour la préparation des nanocapsules de l'invention peuvent également contenir un nombre limité de motifs de ramification dérivés de triols. Les triols utilisés sont généralement choisis parmi le glycérol, le triméthyloléthane et le triméthylolpropane. La fraction des motifs de ramification dérivés des triols ci-dessus n'excède généralement pas 5 % en moles par rapport à l'ensemble des motifs dérivés de diols et de triols.

Selon un mode de réalisation préféré de la présente invention, le polyester polyol formant l'enveloppe des nanocapsules est choisi parmi les polyesters polyols obtenus à partir d'acide adipique, de 1,4-butanediol, et de 1,6-hexane diol et les polyesters polyols obtenus à partir d'acide adipique, de 1,4-butanediol et de 2-éthyl-2-(hydroxyméthyl)-1,3-propanediol. Comme polyesters polyols préférés, on peut citer par exemple ceux commercialisés par la société INOLEX sous les dénominations LEXOREZ et notamment LEXOREZ 1151-35 et LEXOREZ 1460-36. Sont particulièrement préférés, ceux obtenus à partir d'acide adipique, de 1,4-butanediol et de 2-éthyl-2-(hydroxyméthyl)-1,3-propanediol.

Les polyesters polyols utilisés dans la présente invention ont une masse molaire moyenne en poids (mesurée par chromatographie de perméation de gel) inférieure à 5000 et de préférence inférieure à 4000. Leur masse molaire peut ainsi aller par exemple de 1000 à 4500, mieux de 1000 à 4000 et encore mieux de 2000 à 4000. Leur viscosité à 60°C est de préférence inférieure à 6000 cPoises (= 6 Pa.s). Ainsi, le polyester polyol LEXOREZ 1151-35 présente à 60°C une viscosité de 3000 cps et le polyester polyol LEXOREZ 1460-36 présente à 60°C une viscosité de 3500 cps.

Les polyesters polyols utilisés dans la présente invention ont de préférence un point de fusion allant de 30 à 90 °C et mieux de 35 à 70 °C.

Les polyesters polyols utilisés dans la présente invention peuvent être préparés selon des procédés habituellement utilisés pour la préparation des polyesters.

Les polyesters polyols décrits ci-dessus sont utilisés pour préparer des nanocapsules constituées d'un coeur lipidique formant ou contenant un actif lipophile, entouré d'une enveloppe formée par ces polymères.

Le procédé de préparation de nanocapsules, utilisé de préférence par la demanderesse est celui décrit dans le document EP-A-0 274 961, et il comprend les étapes consistant :
- à dissoudre le polymère, la phase lipidique formant ou contenant l'actif et éventuellement un agent d'enrobage dans un solvant organique approprié, c'est-à-dire miscible avec l'eau, non toxique et plus volatil que l'eau (généralement de l'acétone et/ou un alcool inférieur),
- à préparer une solution d'un agent tensioactif approprié dans de l'eau (non-solvant du polymère et de la phase lipidique),
- à verser la phase organique dans la phase aqueuse tout en agitant modérément celle-ci, ce qui aboutit à la formation spontanée d'une émulsion de nanocapsules,
- puis à évaporer la phase organique et, éventuellement, une partie de la phase aqueuse (par exemple à une température de 35 à 40°C), pour obtenir une suspension concentrée de nanocapsules dans une phase aqueuse.

Aussi, l'invention a aussi pour objet un procédé de préparation des nanocapsules selon l'invention, consistant :
- à dissoudre un polymère, une phase lipidique formant ou contenant au moins un actif et éventuellement un agent d'enrobage dans un solvant organique miscible à l'eau approprié,
- à préparer une solution aqueuse d'un agent tensioactif approprié,
- à verser la phase organique dans la phase aqueuse tout en agitant celle-ci, de préférence modérément,
- puis à évaporer la phase organique et, éventuellement, une partie de la phase aqueuse,
caractérisé par le fait que le polymère utilisé dans la première étape est un polyester polyol tel que décrit ci-dessus.

Ce procédé de préparation implique généralement le chauffage de la phase organique et/ou de la phase aqueuse à des températures comprises entre 35 et 70 °C. Les polyesters polyols utilisés dans la présente invention permettent de réaliser ce procédé à température ambiante, ce qui constitue un avantage important en particulier pour des substances actives thermosensibles telles que le rétinol.

Quand l'actif est sensible à l'oxydation et/ou à la chaleur, la préparation des nanocapsules est de préférence réalisée sous une lumière inactinique, sous atmosphère inerte et à température ambiante.

L'agent tensioactif dissous dans la phase aqueuse lors de la préparation des nanocapsules sert principalement à maîtriser la taille des nanocapsules. Il assure en effet la stabilité des nanocapsules dans l'émulsion résultant du versement de la phase acétonique dans la phase aqueuse et prévient leur coalescence. On peut utiliser n'importe quel agent tensioactif à caractère hydrophile, qu'il soit non ionique, anionique ou cationique. On peut citer à titre d'exemple le laurylsulfate de sodium, les composés d'ammonium quaternaire, les monoesters de sorbitanne polyoxyéthylénés ou non, les éthers d'alcools gras et de polyoxyéthylèneglycol, les condensats d'oxyde d'éthylène et d'oxyde de propylène comme les produits PLURONIC® F-68 ou PLURONIC® F-108 vendus par la société BASF, ou les phospholipides tels que la lécithine. Le rapport pondéral de l'agent tensioactif aux matériaux constitutifs des nanocapsules va avantageusement de 0,01 à 0,5 et il est de préférence voisin de 0,2.

Il est souvent souhaitable ou nécessaire de pourvoir les nanocapsules d'un enrobage dit "lamellaire". Il s'agit d'une structure organisée en un ou plusieurs feuillet(s) lipidique(s) constitué(s) chacun d'une bicouche de molécules amphiphiles semblable à celle des membranes biologiques. L'enveloppe polymérique des nanocapsules selon l'invention peut être ainsi entourée d'un enrobage lamellaire ayant une structure organisée en un ou plusieurs feuillet(s) constitué(s) chacun d'une double couche de molécules amphiphiles constituant un agent d'enrobage. Cet enrobage, outre sa fonction d'ajustement de la taille des nanocapsules, améliore l'étanchéité des nanocapsules vis-à-vis d'une fuite de l'actif vers une autre phase lipidique de la composition.

Les agents d'enrobage sont des agents tensioactifs à caractère hydrophobe, solubles dans la phase organique utilisée dans le procédé décrit ci-dessus et qui sont capables, en présence d'eau, de former les doubles couches lipidiques décrites ci-dessus. Dans le procédé d'encapsulation de principes actifs, utilisé par la demanderesse, cet agent d'enrobage est dissous dans la phase organique (acétonique/alcoolique) contenant le polymère et la phase lipidique.

On peut citer à titre d'exemple de tels agents d'enrobage, les phospholipides tels que la lécithine comme décrit dans le document EP-A-447 318; certains polycondensats d'oxyde d'éthylène et d'oxyde de propylène, comme les produits vendus sous la dénomination PLURONIC® par la société BASF tels que PLURONIC® L121 ou sous la dénomination SYNPERONIC® par la société ICI ; ou les agents tensioactifs siliconés (silicones comportant au moins une chaîne oxyéthylénée et/ou oxypropylénée) capables de former des structures lamellaires, tels que ceux décrits dans les documents US-A-5,364,633 et US-A-5,411,744 et utilisés dans la demande de brevet FR-A-2,742,677, par exemple ceux vendus par la société DOW CORNING sous les dénominations DC 5329, DC 7439-146, DC 2-5695 et Q4-3667 ; et leurs mélanges.

La taille moyenne des nanocapsules à base de polyesters polyols ainsi obtenues selon l'invention est généralement inférieure à 1 micron, de préférence inférieure à 500 nm. Elle va avantageusement de 50 à 800 nm, de préférence de 100 à 400 nm. La détermination de cette taille peut être réalisée par exemple à l'aide d'un granulomètre à laser (modèle Amtech BI 90 Plus de la société Broohaven Instrument).

Les nanocapsules de la présente invention peuvent contenir toutes sortes d'actifs lipophiles cosmétiques ou dermatologiques. On peut citer à titre d'exemple d'actifs lipophiles encapsulés, les vitamines et leurs dérivés, les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants, et leurs mélanges.

Selon la présente invention, l'actif lipophile encapsulé est de préférence choisi parmi les actifs lipophiles sensibles aux conditions physico-chimiques environnantes telles que la température, le pH, la lumière ou la présence d'agents oxydants, et en particulier les actifs sensibles à l'oxydation et à l'hydrolyse.

On peut citer à titre d'exemples d'actifs lipophiles préférés, le rétinol (vitamine A) et ses esters, en particulier les esters comportant de 1 à 30 atomes de carbone et plus spécialement les esters comportant de 1 à 10 atomes de carbone, notamment ceux comportant de 1 à 6 atomes de carbone qui sont plus sensibles. On peut citer notamment le propionate de rétinol, l'acétate de rétinol, le butyrate de rétinol, le pivalate de rétinol, le valerate de rétinol, l'hexanoate de rétinol, l'heptanoate de rétinol, le cyclopentane carboxylate de rétinol, le caprate de rétinol, caprylate de rétinol ; et leurs mélanges.

On peut citer aussi comme actifs lipophiles, la vitamine E ou ses esters tels que l'acétate de tocophérol ; la vitamine D ou ses dérivés ; la vitamine F ou ses dérivés ; les carotènes tels que le β-carotène et ses dérivés tels que le lycopène ; l'acide salicylique ou ses dérivés, notamment ceux décrits dans les documents FR-A-2,581,542, EP-A-378 936 et EP-A-570 230, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique; les stéroïdes tels que la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam) ;et leurs mélanges.

Les nanocapsules selon la présente invention se trouvent généralement dans une suspension aqueuse, comme le montre le procédé de préparation décrit ci-dessus. Aussi, l'invention se rapporte aussi à une suspension aqueuse de nanocapsules renfermant, dans un milieu aqueux, des nanocapsules à base de polyesters polyols telles que décrites ci-dessus.

Les nanocapsules selon l'invention et les suspensions aqueuse les contenant peuvent notamment être utilisées dans des compositions à application topique, en particulier des compositions cosmétiques et/ou dermatologiques.

L'invention a également pour objet une composition à application topique, contenant dans un milieu physiologiquement acceptable, une ou plusieurs nanocapsules telles que décrites ci-dessus ou une suspension aqueuse de nanocapsules. La composition peut constituer notamment une composition cosmétique ou dermatologique.

Par milieu physiologiquement acceptable, on entend un milieu compatible avec les matières kératiniques telles que la peau, les muqueuses, les ongles, le cuir chevelu et les cheveux.

La quantité de nanocapsules dans les compositions à application topique de la présente invention va généralement de 0,1 et 30 % en poids et de préférence de 0,5 à 15 % en poids par rapport au poids total de la composition.

Les compositions selon l'invention peuvent se présenter sous toutes les formes habituelles dans le domaine concerné. Elles peuvent se présenter par exemple sous forme d'un sérum, d'une lotion, d'un gel aqueux, hydroalcoolique ou huileux, d'une émulsion eau-dans-huile ou huile-dans-eau ou multiple (E/H/E ou H/E/H), ou encore sous forme d'une dispersion aqueuse de vésicules lipidiques constituées de lipides ioniques ou non-ioniques ou d'un mélange de ceux-ci, lesquelles vésicules renferment ou non une phase huileuse.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme coulée, en coupelle ou sous forme de stick.

Le milieu physiologiquement acceptable peut comprendre une phase aqueuse et/ou une phase huileuse. Il comprend généralement au moins une phase aqueuse puisque les nanocapsules se présentent le plus souvent dans une suspension aqueuse. Cette phase aqueuse des compositions selon l'invention comprend au moins de l'eau. Selon la forme galénique de la composition, la quantité de phase aqueuse va généralement de 40 à 100 % en poids par rapport au poids total de la composition et de préférence de 60 à 95 % en poids par rapport au poids total de la composition. La quantité d'eau peut représenter tout ou une partie de la phase aqueuse et elle est généralement d'au moins 30 % en poids par rapport au poids total de la composition.

Les compositions de l'invention peuvent contenir dans la phase aqueuse ou dans la phase huileuse si elle comporte une phase huileuse, un ou plusieurs solvants organiques, hydrophiles, lipophiles et/ou amphiphiles, physiologiquement acceptables, c'est-à-dire bien tolérés et donnant un toucher cosmétiquement acceptable.

Les solvants organiques peuvent représenter de 0,5 à 50 % et de préférence de 2 à 20 % du poids total de la composition. Les solvants organiques peuvent être choisis dans le groupe constitué par les solvants organiques hydrophiles, les solvants organiques lipophiles, les solvants amphiphiles, ou leurs mélanges.

Parmi les solvants organiques, on peut citer par exemple les mono-alcools inférieurs linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le propylène glycol, le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène, tels que le polyéthylène glycol 32 OE ; les éthers d'éthylène glycol comme le diéthylène glycol mono-méthyl ou mono-éthyl éther ; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alkyle, tels que l'adipate de diisopropyle, l'adipate de dioctyle, les benzoates d'alkyle ; et leurs mélanges.

Quand la composition comporte une phase huileuse, la nature de cette phase huileuse n'est pas critique. La phase huileuse peut ainsi être constituée par tous les corps gras et notamment les huiles, classiquement utilisés dans les domaines cosmétique ou dermatologique. La phase huileuse comprend généralement au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le palmitate d'isocétyle ; le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras comme l'heptanoate de stéaryle ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'huile de vaseline, les polydécènes, le polyisobutène hydrogéné tel que l'huile de Parléam® ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; la vaseline ; les gommes telles que les gommes de silicone (diméthiconol) ; les résines de silicone telles que la trifluorométhyl-C1-4-alkyldimethicone et la trifluoropropyldimethicone.

Ces corps gras peuvent être choisis de manière variée par l'homme du métier afin de préparer une composition ayant les propriétés, par exemple de consistance ou de texture, souhaitées.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt ou le mélange de Cetyl dimethicone copolyol, Polyglyceryl-4 Isostearate et Hexyllaurate ; vendu sous la dénomination Abil WE09^{R} par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) ; le ricinoléate de glycéryle ; le monostéarate de diglycérol ; et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 20 OE (nom CTFA : PEG-20 stearate), et leurs mélanges.

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate); l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesquiisostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel; le cétostéaryl glucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidyl glucoside commercialisé sous la dénomination Montanov 202 par la société Seppic. et leurs mélanges.

La composition peut aussi contenir d'autres tensioactifs, et notamment des tensioactifs anioniques tels que par exemple les acylglutamates mono- et disodiques comme le sel disodique de l'acide N-stéaroyl L-glutamique commercialisé sous la dénomination Acylglutamate HS21 par la société Ajinomoto.

De façon connue, toutes les compositions de l'invention peuvent contenir un ou plusieurs des adjuvants habituels dans les domaines cosmétique et dermatologique, choisis par exemple parmi les agents gélifiants et/ou épaississants (hydrophiles ou lipophiles) ; les émollients ; les actifs (hydrophiles ou lipophiles) autres ou identiques à ceux indiqués ci-dessus ; les agents anti-radicaux libres ; les séquestrants ; les antioxydants ; les conservateurs ; les agents alcanisants ou acidifiants ; les parfums ; les agents filmogènes ; les matières colorantes (pigments tels que les oxydes de fer et le dioxyde de titane, nacres, colorants solubles) ; les charges (par exemple poudre de polyéthylène, poudre de Nylon) ; et leurs mélanges. Les quantités de ces différents adjuvants sont celles classiquement utilisées dans les domaines considérés, généralement de 0,001 à 30 % en poids et de préférence de 0,1 à 20 % en poids par rapport au poids total de la composition.

Comme actifs utilisables dans la composition de l'invention, on peut citer par exemple les agents hydratants tels que les hydrolysats de protéines et les polyols comme la glycérine, les glycols comme les polyéthylène glycols, et les dérivés de sucre; les extraits naturels; les anti-inflammatoires; les oligomères procyannidoliques ; les vitamines comme la vitamine C (acide ascorbique), la vitamine B5 (panthénol), la vitamine B3 (niacinamide), les dérivés de ces vitamines (notamment esters) et leurs mélanges; l'urée; la caféine; les dépigmentants tels que l'acide kojique, l'hydroquinone et l'acide caféique ; les alpha-hydroxyacides tels que l'acide lactique et l'acide glycolique, et leurs dérivés ; les filtres solaires ; l'hydrocortisone ; la mélatonine ; les extraits d'algues, de champignons, de végétaux, de levures, de bactéries; les enzymes; les stéroïdes ; les actifs anti-bactériens comme le 2,4,4'-trichloro-2'-hydroxy diphényl éther (ou triclosan), le 3,4,4'-trichlorocarbanilide (ou triclocarban) ; les bêta-hydroxyacides comme l'acide salicylique et ses dérivés ; les agents matifiants comme les fibres ; les agents tenseurs ; les poudres organiques ou inorganiques, notamment celles ayant une taille de 20 nm à 20 µm ; et leurs mélanges.

Selon la fluidité de la composition que l'on souhaite obtenir, on peut y ajouter un ou plusieurs gélifiants hydrophiles ou lipophiles. Comme gélifiants hydrophiles, on peut citer par exemple les polymères carboxyvinyliques, tels que les carbomers ; les polyacrylamides et les polymères et copolymères d'acide 2-acrylamido 2-méthylpropane sulfonique), éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide).

Comme gélifiants lipophiles, on peut citer les argiles modifiées telles que les bentones telles que le mélange « cyclomethicone, Quaternium-18 hectorite, SD alcohol 40 » (10/85/5) (nom CTFA) commercialisé sous la dénomination Bentone Gel VS-5 par la société Rheox ; les organopolysiloxanes élastomères réticulés tels que ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels: KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric.

Ces gélifiants, lorsqu'ils sont présents, sont généralement utilisés à des concentrations allant de 0,01 à 10 % et de préférence de 0,05 à 5 % en poids de matière active par rapport au poids total de la composition.

Bien entendu, l'homme de métier veillera à choisir ce ou ces éventuels composés supplémentaires et leur quantité de manière à ce que les propriétés avantageuses attachées intrinsèquement à la composition cosmétique ou dermatologique conforme à l'invention ne soient pas, ou substantiellement pas, altérées par la ou les adjonctions envisagées.

La composition contenant les nanocapsules présente de préférence un pH qui respecte la peau et qui va généralement de 3 à 8 et de préférence de 4,5 à 7.

Les compositions selon l'invention peuvent être utilisées pour toute application appropriée selon l'actif encapsulé.

Ainsi, les compositions de l'invention peuvent être utilisées comme produit de soin (ou de traitement), de protection, de nettoyage, de démaquillage et /ou de maquillage des matières kératiniques (peau, cheveux, cuir chevelu, cils, sourcils, ongles ou muqueuses) tels que des crèmes de protection, de traitement ou de soin pour le visage, pour les mains ou pour le corps, des laits corporels de protection ou de soin, des gels ou mousses pour le soin de la peau et/ou des muqueuses (lèvres).

Les compositions de l'invention contenant des filtres solaires peuvent être également utilisées comme produit de protection solaire.

Les compositions de l'invention peuvent aussi être utilisées comme produits pour le maquillage, notamment le maquillage de la peau, des sourcils, des cils et des lèvres. Les produits de maquillage sont le plus souvent colorés et contiennent généralement des pigments. Sous forme de produits de maquillage, les compositions de l'invention peuvent constituer avantageusement un fond de teint, un rouge à lèvres, un fard à joues, un fard à paupières, un mascara ou un eyeliner.

Les compositions selon l'invention peuvent être également utilisées comme produits rincés ou comme produits non-rincés pour le nettoyage de la peau du visage et/ou du corps et/ou pour le nettoyage des cheveux, par exemple comme produits capillaires y compris pour le soin et le conditionnement des cheveux.

L'invention a aussi pour objet l'utilisation cosmétique d'une composition cosmétique telle que définie ci-dessus, comme produit de soin, de nettoyage et/ou de démaquillage de la peau, des cheveux, du cuir chevelu, des cils, des sourcils, des ongles ou des muqueuses.

L'invention a aussi pour objet l'utilisation cosmétique d'une composition cosmétique telle que définie ci-dessus, comme produit de maquillage.

L'invention a aussi pour objet l'utilisation cosmétique d'une composition cosmétique telle que définie ci-dessus, comme produit de protection solaire (protection contre le soleil et/ou les U.V. des appareils à bronzer).

L'invention a aussi pour objet l'utilisation cosmétique d'une composition cosmétique telle que définie ci-dessus, comme produit capillaire rincé ou non-rincé.

Un autre objet de l'invention est un procédé de traitement cosmétique (non-thérapeutique) d'une matière kératinique (peau, cuir chevelu, cheveux, cils, sourcils, ongles ou muqueuses), caractérisé en ce qu'on applique sur la matière kératinique, une composition cosmétique telle que définie ci-dessus. La matière kératinique est notamment la peau.

Les exemples, donnés ci-après à titre purement illustratif et non limitatif, permettront de mieux comprendre l'invention. Les quantités y sont indiquées en pourcentage en poids sauf mention contraire.

### Exemple 1 : Préparation de nanocapsules de propionate de rétinol

Dans un ballon en verre ambré d'une capacité de 250 ml, on dissout, sous atmosphère inerte, à température ambiante et sous agitation, dans 100 ml d'acétone :
- 1 g du polyester polyol à base d'acide adipique, de butane diol et de 2-ethyl-2(hydroxymethyl)-1,3-propanediol, commercialisé sous la dénomination LEXOREZ 1151-35 par la société INOLEX, et
- 0,5 g de DC2-5695 (Polydimethylsiloxane oxyéthyléné) commercialisé par la société Dow Corning.

A la solution obtenue, on ajoute 2,5 g de propionate de rétinol.

D'autre part, dans un ballon en verre ambré d'une capacité de 500 ml, on dissout sous atmosphère inerte et à température ambiante, sous agitation, 0,25 g de Pluronic F108 (ou Poloxamer 338) (128 OE / 54 OP / 128 OE) commercialisé par la société BASF, dans 150 g d'eau.

On verse la phase acétonique dans la phase aqueuse en maintenant l'agitation. Puis, on évapore ensuite dans un évaporateur rotatif jusqu'à obtention d'un volume final de 50 ml.

Cette suspension aqueuse contient des nanocapsules (enrobées) ayant un diamètre moyen de 263 nm.

### Exemple 2 : Préparation de nanocapsules de propionate de rétinol

On procède de la manière décrite dans l'Exemple 1 mais en remplaçant le polyester polyol à base d'acide adipique, de butane diol et de 2-éthyl-2-(hydroxyméthyl)-1,3-propanediol par une quantité identique en poids de polyester polyol à base d'acide adipique, de butane diol, d'hexane diol, commercialisé sous la dénomination LEXOREZ 1460-36 par la société INOLEX.

On obtient une suspension aqueuse de nanocapsules (enrobées) ayant un diamètre moyen de 251 nm.

### Exemple 3 : Essais de stabilité du rétinol encapsulé dans différents polymères

On compare la stabilité du propionate de rétinol enfermé dans les nanocapsules selon les exemples 1 et 2, à la stabilité de cet actif dans des nanocapsules à base de poly(éthylène)adipate (nommé ci-après PEA) ayant un poids moléculaire de 10 000, commercialisé par la société Scientific Polymer Product, et à base de Capa 656 (polycaprolactone ayant un PM de 56 000 et commercialisée par la société Solvay), toutes ces nanocapsules étant enrobées de l'agent siliconé DC-5695.

Les nanocapsules contenant le propionate de rétinol sont conservées sous forme de suspension aqueuse pendant un mois à 45°C en conditionnements clos et étanches à la lumière et aux gaz. Au bout de cette période de conservation, on évalue la perte en principe actif (propionate de rétinol) par HPLC.

Les résultats obtenus sont rassemblés dans le tableau suivant :

| Polymère | PEA | Capa 656 | Lexorez 1151-35(1) | Lexorez 1460-36 (2) |
|---|---|---|---|---|
| Masse molaire | 10 000 | 50 000 | 3200 | 3200 |
| Point de fusion en °C | 55 | 58-60 | 55-65 | 40-50 |
| Agent d'enrobage | Silicone DC-5695 | Silicone DC-5695 | Silicone DC-5695 | Silicone DC-5695 |
| Diamètre des capsules | 210 nm | 269 nm | 263 nm | 251 nm |
| pH de la suspension | 7,0 | 8,2 | 8,1 | 7,9 |
| % de perte après 1 mois à 45°C | 31% | 26% | 15% | 12% |

| | | | | |
|---|---|---|---|---|
| (1) Lexorez 1151-35: butanediol/2-ethyl-2-(hydroxymethyl)-1,3-propanediol/acide adipique | | | | |
| (2) Lexorez 1460-36 : butanediol/hexanediol/acide adipique | | | | |

Ces résultats montrent que l'utilisation de polyesters polyols du type Lexorez, qui sont obtenus à partir de deux alcane-diols ou d'un alcane-diol et d'un hydroxyalkyl alcane-diol et qui ont une masse molaire inférieure à 5000, améliore la stabilité du propionate de vitamine A encapsulé.

### Exemple 4 : Emulsion H/E

| *Phase Huileuse :* | |
|---|---|
| Monostéarate de diglycérol | 2,0 % |
| PEG-20 stearate | 1,5 % |
| N Stearoyl L glutamique acide disodique (Acyl glutamate HS21 de la société Ajinomoto) | 0,5 % |
| Huile de vaseline | 3 % |
| Vaseline | 1 % |
| Stéaryl Heptanoate | 3 % |
| Huile d'amande d'abricot | 5 % |
| Polyisobutène hydrogéné | 5 % |
| Isocétyl palmitate | 2 % |
| Silicone volatile | 5 % |
| Vitamine E | 0,5 % |
| Conservateur | 0,3 % |

| *Phase aqueuse* 1 | |
|---|---|
| Glycérol | 5 % |
| Conservateurs | 1 % |
| Eau distillée | Qsp 100 % |

| *Phase aqueuse* 2 | |
|---|---|
| Carbomer | 0,4 % |
| Eau distillée | 15 % |
| Conservateurs | 0,1 % |
| Triéthanolamine | 0,4 % |
| | |

| *Phase aqueuse* 3 | |
|---|---|
| Nanocapsules de propionate de rétinol selon l'exemple 2 | |
| dosée à 2.5% de propionate de rétinol | 10 % |

Mode opératoire : La phase aqueuse 1 est introduite à 60°C dans la phase huileuse elle même à cette température, sous agitation très vive. On maintient la température et l'agitation pendant 30 minutes. La suspension est ensuite amenée à température ambiante. La phase aqueuse 2 est alors dispersée à l'aide d'un disperseur non cisaillant. Puis, la phase aqueuse 3 (suspension de nanocapsules) est ensuite introduite sous agitation légère.

On obtient une crème de jour pour le soin de la peau, contre les signes de l'âge.

## Revendications

1. Nanocapsule constituée :
- d'un coeur lipidique formant ou contenant au moins un actif lipophile et
- d'une enveloppe polymérique continue insoluble dans l'eau, comprenant au moins un polyester polyol obtenu par polycondensation d'au moins un acide dicarboxylique aliphatique avec au moins deux alcane-diols ou avec au moins un alcane-diol et au moins un hydroxyalkyl alcane-diol comportant éventuellement une chaîne alkyle, le polyester polyol ayant une masse molaire moyenne en poids inférieure à 5000.

2. Nanocapsule selon la revendication 1, **caractérisée par le fait que** l'acide dicarboxylique aliphatique est choisi parmi l'acide malonique, l'acide succinique, l'acide glutarique, l'acide adipique, l'acide pinélique, l'acide sébacique, l'acide azélaique, et leurs mélanges.

3. Nanocapsule selon la revendication précédente, **caractérisée par le fait que** l'acide dicarboxylique aliphatique est l'acide adipique.

4. Nanocapsule selon l'une quelconque des revendications précédentes, **caractérisée par le fait que** l'alcane-diol est choisi parmi les alcane-diols à chaîne linéaire ou ramifiée comportant de 2 à 20 atomes de carbone, et de préférence de 2 à 10 atomes de carbone.

5. Nanocapsule selon la revendication précédente, **caractérisées par le fait que** l'alcane-diol est choisi parmi l'éthylèneglycol, le propylèneglycol, le 1,3-propanediol, le 1,4-butanediol, le 1,5-pentanediol, le 1,6-hexanediol, le néopentylglycol, et leurs mélanges.

6. Nanocapsule selon l'une quelconque des revendications précédentes, **caractérisée par le fait que** l'hydroxyalkyl alcane-diol est choisi parmi les 2-alkyl-2-(hydroxyalkyl)-1,3-propanediol, où le groupe hydroxyalkyle et la chaîne alkyle comportent, indépendamment l'un de l'autre, de 1 à 10 atomes de carbone ; les 2-(hydroxyalkyl)-1,3-propanediol où le groupe hydroxyalkyle comporte de 1 à 10 atomes de carbone ; et leurs mélanges.

7. Nanocapsule selon l'une quelconque des revendications précédentes, **caractérisée par le fait que** le polyester polyol est choisi parmi les polyesters polyols obtenus à partir d'acide adipique, de 1,4-butanediol, et de 1,6-hexane diol et les polyesters polyols obtenus à partir d'acide adipique, de 1,4-butanediol et de 2-ethyl-2-(hydroxymethyl)-1,3-propanediol.

8. Nanocapsule selon l'une des revendications précédentes, **caractérisée par le fait que** les polyesters polyols ont une masse molaire moyenne en poids allant de 1000 à 4500, de préférence de 1000 à 4000.

9. Nanocapsule selon l'une des revendications précédentes, **caractérisée par le fait que** les polyesters polyols ont un point de fusion allant de 30 à 90°C.

10. Nanocapsule selon l'une quelconque des revendications précédentes, **caractérisée par le fait que** l'enveloppe polymérique est entourée d'un enrobage lamellaire ayant une structure organisée en un ou plusieurs feuillet(s) constitué(s) chacun d'une double couche de molécules amphiphiles constituant un agent d'enrobage.

11. Nanocapsule selon la revendication précédente, **caractérisée par le fait que** ledit agent d'enrobage est choisi parmi les phospholipides ; les polycondensats d'oxyde de propylène et d'oxyde d'éthylène et les agents tensioactifs siliconés capables de former des structures lamellaires ; et leurs mélanges.

12. Nanocapsule selon l'une quelconque des revendications précédentes, **caractérisée par le fait qu'**elle a une taille moyenne allant de 50 nm à 800 nm, de préférence de 100 nm à 400 nm.

13. Nanocapsule selon l'une quelconque des revendications précédentes, **caractérisée par le fait que** l'actif lipophile est choisi parmi les vitamines et leurs dérivés, les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants, et leurs mélanges.

14. Nanocapsule selon la revendication précédente, **caractérisée par le fait que** l'actif lipophile est choisi parmi le rétinol et ses esters comportant de 1 à 30 atomes de carbone, plus particulièrement de 1 à 10 atomes de carbone ; et leurs mélanges.

15. Nanocapsule selon la revendication précédente, **caractérisées par le fait que** l'actif lipophile est choisi parmi le propionate de rétinol, l'acétate de rétinol, le butyrate de rétinol, le pivalate de rétinol, le valerate de rétinol, l'hexanoate de rétinol, l'heptanoate de rétinol, le cyclopentane carboxylate de rétinol, le caprate de rétinol, le caprylate de rétinol ; et leurs mélanges.

16. Nanocapsule selon l'une quelconque des revendications précédentes, **caractérisée par le fait que** l'actif lipophile est choisi parmi la vitamine E ou ses esters ; la vitamine D ou ses dérivés; la vitamine F ou ses dérivés; les carotènes ; l'acide salicylique ou ses dérivés ; les stéroïdes ; et leurs mélanges.

17. Suspension aqueuse de nanocapsules renfermant, dans un milieu aqueux, des nanocapsules à base de polyesters polyols selon l'une quelconque des revendications 1 à 16.

18. Composition à application topique, **caractérisée par le fait qu'**elle contient, dans un support physiologiquement acceptable, une ou plusieurs nanocapsules selon l'une quelconque des revendications 1 à 16 ou la suspension de nanocapsules selon la revendication 17.

19. Composition selon la revendication précédente, **caractérisée par le fait qu'**elle contient de 0,1 à 30 % en poids et de préférence de 0,5 à 15 % en poids de nanocapsules, par rapport au poids total de la composition.

20. Composition selon la revendication 18 ou 19, **caractérisée par le fait qu'**elle se présente sous forme d'un sérum, d'une lotion, d'un gel aqueux, hydroalcoolique ou huileux, d'une émulsion eau-dans-huile ou huile-dans-eau, ou encore sous forme d'une dispersion aqueuse de vésicules lipidiques constituées de lipides ioniques ou non-ioniques ou d'un mélange de ceux-ci, lesquelles vésicules renferment ou non une phase huileuse.

21. Composition selon l'une quelconque des revendications 18 à 20, **caractérisée par le fait qu'**elle constitue une composition cosmétique ou dermatologique.

22. Procédé de préparation des nanocapsules selon l'une quelconque des revendications 1 à 16, consistant :
- à dissoudre un polymère, une phase lipidique formant ou contenant au moins un actif et éventuellement un agent d'enrobage dans un solvant organique miscible à l'eau approprié,
- à préparer une solution aqueuse d'un agent tensioactif approprié,
- à verser la phase organique dans la phase aqueuse tout en agitant celle-ci,
- puis à évaporer la phase organique et, éventuellement, une partie de la phase aqueuse,
procédé **caractérisé par le fait que** le polymère utilisé dans la première étape est un polyester polyol selon l'une quelconque des revendications 1 à 9.

23. Utilisation cosmétique d'une composition cosmétique selon l'une quelconque des revendications 18 à 20, comme produit de soin, de nettoyage et/ou de démaquillage de la peau, des cheveux, du cuir chevelu, des cils, des sourcils, des ongles ou des muqueuses, ou comme produit de maquillage ou comme produit de protection solaire.

24. Procédé de traitement cosmétique d'une matière kératinique, notamment la peau, **caractérisé par le fait qu'**on applique sur la matière kératinique, une composition cosmétique selon l'une quelconque des revendications 18 à 20.

## Patentansprüche

1. Nanokapsel bestehend aus:
- einem Lipidkern, der aus mindestens einem lipophilen Wirkstoff gebildet wird oder mindestens einen lipophilen Wirkstoff enthält, und
- einer kontinuierlichen, in Wasser unlöslichen Polymerschale, die mindestens ein Polyesterpolyol enthält, das durch Polykondensation mindestens einer aliphatischen Dicarbonsäure mit mindestens zwei Alkandiolen oder mit mindestens einem Alkandiol und mindestens einem Hydroxyalkylalkandiol, das gegebenenfalls eine Alkylkette aufweist, erhalten wird, wobei das Polyesterpolyol eine gewichtsmittlere Molmasse unter 5 000 aufweist.

2. Nanokapsel nach Anspruch 1, **dadurch gekennzeichnet, dass** die aliphatische Dicarbonsäure unter Malonsäure, Bernsteinsäure, Glutarsäure, Adipinsäure, Pimelinsäure, Sebacinsäure, Azelainsäure und deren Gemischen ausgewählt ist.

3. Nanokapsel nach dem vorhergehenden Ansprüche, **dadurch gekennzeichnet, dass** die aliphatische Dicarbonsäure die Adipinsäure ist.

4. Nanokapsel nach einem der vorhergehenden Ansprüche, **dadurch gekennzeichnet, dass** das Alkandiol unter den Alkandiolen mit gerader oder verzweigter Kette ausgewählt ist, die 2 bis 20 Kohlenstoffatome und vorzugsweise 2 bis 10 Kohlenstoffatome enthalten.

5. Nanokapsel nach dem vorhergehenden Anspruch, **dadurch gekennzeichnet, dass** das Alkandiol unter Ethylengtykol, Propylenglykol, 1,3-Propandiol, 1,4-Butandiol, 1,5-Pentandiol, 1,6-Hexandiol, Neopentylglykol und deren Gemischen ausgewählt ist.

6. Nanokapsel nach einem der vorhergehenden Ansprüche, **dadurch gekennzeichnet, dass** das Hydroxyalkylalkandiol unter den 2-Alkyl-2-(hydroxyalkyl)-1,3-propandiolen, bei denen die Hydroxygruppe und die Alkylgruppe unabhängig voneinander 1 bis 10 Kohlenstoffatome aufweisen; den 2-(Hydroxyalkyl)-1,3-propandiolen, bei denen die Hydroxyalkylgruppe 1 bis 10 Kohlenstoffatome aufweist; und deren Gemischen ausgewählt ist.

7. Nanokapsel nach einem der vorhergehenden Ansprüche, **dadurch gekennzeichnet, dass** das Polyesterpolyol unter den Polyesterpolyolen, die ausgehend von Adipinsäure, 1,4-Butandiol und 1,6-Hexandiol erhalten werden, und den Polyesterpolyolen ausgewählt ist, die ausgehend von Adipinsäure, 1,4-Butandiol und 2-Ethyl-2-(hydroxymethyl)-1,3-propandiol hergestellt werden.

8. Nanokapsel nach einem der vorhergehenden Ansprüche, **dadurch gekennzeichnet, dass** die Polyesterpolyole eine gewichtsmittlere Molmasse von 1 000 bis 4 500 und vorzugsweise 1 000 bis 4 000 aufweisen.

9. Nanokapsel nach einem der vorhergehenden Ansprüche, **dadurch gekennzeichnet, dass** die Polyesterpolyole einen Schmelzpunkt von 30 bis 90 °C besitzen.

10. Nanokapsel nach einem der vorhergehenden Ansprüche, **dadurch gekennzeichnet, dass** die Polymerhülle von einer lamellaren Umhüllung mit einer geordneten Struktur aus einer oder mehreren Schicht(en) umhüllt ist, welche jeweils aus einer Doppelschicht aus amphiphilen Molekülen besteht (bestehen), die ein Umhüllungsmittel darstellt.

11. Nanokapsel nach dem vorhergehenden Anspruch, **dadurch gekennzeichnet, dass** das Umhüllungsmittel unter den Phospholipiden; Polykondensaten von Propylenoxid und Ethylenoxid und den grenzflächenaktiven Siliconen, die lamellare Strukturen ausbilden können; und deren Gemischen ausgewählt ist.

12. Nanokapsel nach einem der vorhergehenden Ansprüche, **dadurch gekennzeichnet, dass** sie eine mittlere Größe von 50 bis 800 nm und vorzugsweise 100 bis 400 nm aufweist.

13. Nanokapsel nach einem der vorhergehenden Ansprüche, **dadurch gekennzeichnet, dass** der lipophile Wirkstoff unter den Vitaminen und deren Derivaten, Emollientien, entzündungshemmenden Wirkstoffen, antibakteriellen Wirkstoffen, fungiziden Wirkstoffen, antiviralen Wirkstoffen, Antiseborrhoeika, Wirkstoffen gegen Akne, Keratolytika, Antihistaminika, anästhesierenden Wirkstoffen, Wundheilungsmitteln, die Pigmentierung modifizierenden Wirkstoffen, Sonnenschutzfiltern, Radikalfängern für freie Radikale, Hydratisierungsmitteln und deren Gemischen ausgewählt ist.

14. Nanokapsel nach dem vorhergehenden Anspruch, **dadurch gekennzeichnet, dass** der lipophile Wirkstoff unter Retinol und seinen Estern mit bis 30 Kohlenstoffatomen und insbesondere 1 bis 10 Kohlenstoffatomen und deren Gemischen ausgewählt ist.

15. Nanokapsel nach dem vorhergehenden Anspruch, **dadurch gekennzeichnet, dass** der lipophile Wirkstoff unter Retinolpropionat, Retinolacetat, Retinolbutyrat, Retinolpivalat, Retinolvalerat, Retinolhexanoat, Retinolheptanoat, Retinolcyclopentancarboxylat, Retinolcaprat, Retinolcaprylat und deren Gemischen ausgewählt ist.

16. Nanokapsel nach einem der vorhergehenden Ansprüche, **dadurch gekennzeichnet, dass** der lipophile Wirkstoff unter Vitamin E oder seinen Estern; Vitamin D oder seinen Derivaten; Vitamin F oder seinen Derivaten; Carotinen; Salicylsäure und ihren Derivaten; Steroiden; und deren Gemischen ausgewählt ist.

17. Wässrige Suspension von Nanokapseln, die in einem wässrigen Medium Nanokapseln auf der Basis von Polyesterpolyolen nach einem der Ansprüche 1 bis 16 enthält.

18. Zusammensetzung zur topischen Anwendung, **dadurch gekennzeichnet, dass** sie in einem physiologisch akzeptablen Träger eine oder mehrere Nanokapseln nach einem der Ansprüche 1 bis 16 oder die Suspension von Nanokapseln nach Anspruch 17 enthält.

19. Zusammensetzung nach dem vorhergehenden Anspruch, **dadurch gekennzeichnet, dass** sie 0,1 bis 30 Gew.-% und vorzugsweise 0,5 bis 15 Gew.-% Nanokapseln, bezogen auf das Gesamtgewicht der Zusammensetzung, enthält.

20. Zusammensetzung nach Anspruch 18 oder 19, **dadurch gekennzeichnet, dass** sie als Serum, Lotion, wässriges, wässrig-alkoholisches oder öliges Gel, Wasser-in-Öl-Emulsion, Öl-in-Wasser-Emulsion oder in Form einer wässrigen Dispersion von Lipidvesikeln vorliegt, die aus ionischen oder nichtionischen Lipiden oder einem Gemisch solcher Lipide bestehen, wobei die Vesikel gegebenenfalls eine wässrige Phase enthalten.

21. Zusammensetzung nach einem der Ansprüche 18 bis 20, **dadurch gekennzeichnet, dass** es sich um eine kosmetische oder dermatologische Zusammensetzung handelt.

22. Verfahren zur Herstellung von Nanokapseln nach einem der Ansprüche 1 bis 16, das darin besteht:
- ein Polymer, eine Lipidphase, die mindestens einen Wirkstoff enthält oder bildet, und gegebenenfalls ein Mittel zum Umhüllungen in einem geeigneten, mit Wasser mischbaren, organischen Lösungsmittel zu lösen,
- eine wässrige Lösung eines geeigneten grenzflächenaktiven Stoffes herzustellen,
- die organische Phase unter Rühren in die wässrige Phase zu gießen, und
- die organische Phase und gegebenenfalls einen Teil der wässrigen Phase zu verdampfen,
wobei das Verfahren **dadurch gekennzeichnet ist, dass** das in dem ersten Schritt verwendete Polymer ein Polyesterpolyol nach einem der Ansprüche 1 bis 9 ist.

23. Kosmetische Verwendung einer kosmetischen Zusammensetzung nach einem der Ansprüche 18 bis 20 als Produkt zur Pflege, zur Reinigung und/oder zum Abschminken der Haut, der Haare, der Kopfhaut, der Wimpern, der Augenbrauen, der Nägel oder der Schleimhäute oder als Produkt zum Schminken oder als Produkt zum Sonnenschutz.

24. Verfahren zur kosmetischen Behandlung einer Keratinsubstanz und insbesondere der Haut, **dadurch gekennzeichnet, dass** auf die Keratinsubstanz eine kosmetische Zusammensetzung nach einem der Ansprüche 18 bis 20 aufgetragen wird.

## Claims

1. Nanocapsule consisting of:
- a lipid core forming or containing at least one lipophilic active agent, and
- a water-insoluble continuous polymer envelope, comprising at least one polyester polyol obtained by polycondensation of at least one aliphatic dicarboxylic acid with at least two alkanediols or with at least one alkanediol and at least one hydroxyalkyl alkanediol optionally comprising an alkyl chain, the polyester polyol having a weight-average molar mass of less than 5 000.

2. Nanocapsule according to Claim 1, **characterized in that** the aliphatic dicarboxylic acid is chosen from malonic acid, succinic acid, glutaric acid, adipic acid, pinelic acid, sebacic acid and azelaic acid, and mixtures thereof.

3. Nanocapsule according to the preceding claim, **characterized in that** the aliphatic dicarboxylic acid is adipic acid.

4. Nanocapsule according to any one of the preceding claims, **characterized in that** the alkanediol is chosen from alkanediols with a linear or branched chain containing from 2 to 20 carbon atoms and preferably from 2 to 10 carbon atoms.

5. Nanocapsule according to the preceding claim, **characterized in that** the alkanediol is chosen from ethylene glycol, propylene glycol, 1,3-propanediol, 1,4-butanediol, 1,5-pentanediol, 1,6-hexanediol and neopentyl glycol, and mixtures thereof.

6. Nanocapsule according to any one of the preceding claims, **characterized in that** the hydroxyalkyl alkanediol is chosen from 2-alkyl-2-(hydroxyalkyl)-1,3-propanediols, in which the hydroxyalkyl group and the alkyl chain contain, independently of each other, from 1 to 10 carbon atoms; 2-(hydroxyalkyl)-1,3-propanediols, in which the hydroxyalkyl group contains from 1 to 10 carbon atoms; and mixtures thereof.

7. Nanocapsule according to any one of the preceding claims, **characterized in that** the polyester polyol is chosen from polyester polyols obtained from adipic acid, 1,4-butanediol and 1,6-hexanediol and the polyester polyols obtained from adipic acid, 1,4-butanediol and 2-ethyl-2-(hydroxymethyl)-1,3-propanediol.

8. Nanocapsule according to one of the preceding claims, **characterized in that** the polyester polyols have a weight-average molar mass ranging from 1 000 to 4 500 and preferably from 1 000 to 4 000.

9. Nanocapsule according to one of the preceding claims, **characterized in that** the polyester polyols have a melting point ranging from 30 to 90°C.

10. Nanocapsule according to any one of the preceding claims, **characterized in that** the polymer envelope is surrounded with a lamellar coating whose structure is organized as one or more leaflet(s) each consisting of a double layer of amphiphilic molecules constituting a coating agent.

11. Nanocapsule according to the preceding claim, **characterized in that** the said coating agent is chosen from phospholipids; polycondensates of propylene oxide and of ethylene oxide, and silicone surfactants capable of forming lamellar structures; and mixtures thereof.

12. Nanocapsule according to any one of the preceding claims, **characterized in that** it has a mean size ranging from 50 nm to 800 nm and preferably from 100 nm to 400 nm.

13. Nanocapsule according to any one of the preceding claims, **characterized in that** the lipophilic active agent is chosen from vitamins and derivatives thereof, emollients, anti-inflammatory agents, antibacterial agents, antifungal agents, antiviral agents, anti-seborrhoeic agents, anti-acne agents, keratolytic agents, antihistamines, anaesthetics, cicatrizing agents, pigmentation modifiers, sunscreens, free-radical traps and moisturizers, and mixtures thereof.

14. Nanocapsule according to the preceding claim, **characterized in that** the lipophilic active agent is chosen from retinol and its esters containing from 1 to 30 carbon atoms and more particularly from 1 to 10 carbon atoms; and mixtures thereof.

15. Nanocapsule according to the preceding claim, **characterized in that** the lipophilic active agent is chosen from retinyl propionate, retinyl acetate, retinyl butyrate, retinyl pivalate, retinyl valerate, retinyl hexanoate, retinyl heptanoate, retinyl cyclopentanecarboxylate, retinyl caprate and retinyl caprylate; and mixtures thereof.

16. Nanocapsule according to any one of the preceding claims, **characterized in that** the lipophilic active agent is chosen from vitamin E or its esters; vitamin D or its derivatives; vitamin F or its derivatives; carotenes; salicylic acid or its derivatives; steroids; and mixtures thereof.

17. Aqueous suspension of nanocapsules containing, in an aqueous medium, nanocapsules based on polyester polyols according to any one of Claims 1 to 16.

18. Composition for topical application, **characterized in that** it contains, in a physiologically acceptable support, one or more nanocapsules according to any one of Claims 1 to 16 or the nanocapsule suspension according to Claim 17.

19. Composition according to the preceding claim, **characterized in that** it contains from 0.1% to 30% by weight and preferably from 0.5% to 15% by weight of nanocapsules, relative to the total weight of the composition.

20. Composition according to Claim 18 or 19, **characterized in that** it is in the form of a serum, a lotion, an aqueous, aqueous-alcoholic or oily gel, a water-in-oil or oil-in-water emulsion, or alternatively in the form of an aqueous dispersion of lipid vesicles consisting of ionic or nonionic lipids or of a mixture thereof, these vesicles optionally containing an oily phase.

21. Composition according to any one of Claims 18 to 20, **characterized in that** it constitutes a cosmetic or dermatological composition.

22. Process for preparing the nanocapsules according to any one of Claims 1 to 16, which consists:
- in dissolving a polymer, a lipid phase forming or containing at least one active agent and optionally a coating agent in a suitable water-miscible organic solvent,
- in preparing an aqueous solution of a suitable surfactant,
- in adding the organic phase to the aqueous phase while stirring,
- and then in evaporating the organic phase and, possibly, some of the aqueous phase,
this process being **characterized in that** the polymer used in the first step is a polyester polyol according to any one of Claims 1 to 9.

23. Cosmetic use of a cosmetic composition according to any one of Claims 18 to 20, as a care, cleansing and/or makeup-removing product for the skin, the hair, the scalp, the eyelashes, the eyebrows, the nails or mucous membranes, or as a makeup product or as an antisun product.

24. Cosmetic process for treating a keratin material, in particular the skin, **characterized in that** a cosmetic composition according to any one of Claims 18 to 20 is applied to the keratin material.
